# EUROPEAN PATENT APPLICATION

(11) **EP 3 235 441 A1**
(43) Date of publication of application: **25.10.2017**
(21) Application number: 16166207.7
(22) Date of filing: 20.04.2016
(51) Int. Cl.: A61B 10/00, B01L 3/00, B65D 81/32

(54) **DISPENSING UNIT WITH PREDETERMINED SEQUENCE OF ROTATION**

(71) Applicant: Absolute Liquid Solutions ApS, 7100 Vejle (DK)
(72) Inventor: Langhoff, Sebastian André Jensen, 9000 Aalborg (DK); Høgsaa, Asger Hangstrup, 9000 Aalborg (DK); Høgsaa, Bjarke Hangstrup, 9000 Aalborg (DK); Videbæk, Karsten, 4040 Jyllinge (DK); Jensen, Per Rosenberg, 4684 Holmegaard (DK); Andersen, Bjarne, 3600 Frederikssund (DK)
(74) Representative: Plougmann Vingtoft a/s

(57) **Abstract**

The present invention relates to a dispensing unit (3) for dispensing a substance into an external container (2). The dispensing unit (3) comprises a substance storage (4) with at least one compartment (11) and a connector part (5) in mutual rotational engagement with the substance storage (4). For at least one mutual rotational orientation of the connector part (5) and the substance storage (4), substance can flow from the at least one compartment (11) and into the external container (2) via the dispensing outlet (9) of the connector part (5). For at least one different mutual rotational orientation, substance cannot flow out of the compartment (11). The substance storage (4) and the connector part (5) comprise mutually engagable first and second engagement means (17,18), respectively, which are designed and arranged to ensure that the mutual rotation of the connector part (5) and the substance storage (4) follows a predetermined sequence of mutual rotations between stable mutual rotational orientations.

## Description

### FIELD OF THE INVENTION

The present invention relates to a dispensing unit for dispensing substance prestored in the dispensing unit into an external container, and in particular a dispensing unit wherein the dispensing involves mutual rotation of parts of the dispensing unit relative to each other in order to perform the dispensing.

### BACKGROUND OF THE INVENTION

Preservation of human or animal tissue samples for analysis is traditionally performed by the use of toxic preservation fluids which have to be treated with special care. At the same time, a rapid immersion of the tissue sample is essential for maintaining as many of the characteristic tissue sample properties as possible for structural and molecular analysis. It is therefore important to be particularly attentive to the handling of the toxic preservation fluid, since there is a risk that a potentially hasty use of toxic fluids may give stressful and dangerous situations. Prolonged or repeated contact with preservation fluids may result in development of respiratory impairment or chronic respiratory inflammations such as asthma or chronic bronchitis. Some preservation fluids, such as Formalin, have even been classified as being both toxic and carcinogenic. A single accidental spill involving an exposure to a high concentration of preservation fluid may also result in respiratory impairment due to the toxic fumes that some preservation fluids produce. Furthermore, there is also a risk of eye damages if a person gets the preservation fluid in the eyes. Therefore, the preservation fluids are traditionally handled using a fume cupboard, gloves, and goggles or other types of complete eye protection. If the surgery room does not contain a fume cupboard, the tissue samples taken during surgery need to be transported to another room or location which results in a delay and an extra need for cleaning of the person involved. These factors are time consuming and prolong the time for immersing the tissue sample into a preservation fluid; such delay is disadvantageous as it may cause damage to the tissue sample. Furthermore, not all medical clinics have a fume cupboard, and this may hinder the clinics in being able to perform even small biopsies, such as skin biopsies, due to the toxic danger of the preservation fluid; or it may force some clinics to perform the biopsies nevertheless which involves a great risk.

WO 2013/091640 discloses a dispensing unit aimed at solving the above-mentioned problems by providing a device with which it is possible to handle the preservation fluid without the risk of having contact with it. The inventors of the present invention have found that at least for some applications, the dispensing unit as disclosed in this reference could advantageously be improved. In particular, it would be advantageous to be able to ensure, and possibly monitor, that the dispensing unit is used as intended also when used by inexperienced users.

### OBJECT OF THE INVENTION

It is an object of the present invention to provide a dispensing unit with which it is possible to handle a substance, such as a preservation fluid, without the risk of having physical contact with the substance.

It is another object of the present invention to provide a dispensing unit with which it can be ensured that a predetermined procedure is correctly followed.

It is an object of at least some embodiments of the invention to provide a dispensing unit with which the risk of discrepancy with a predetermined procedure is minimized.

It is another object of at least some embodiments of the invention to provide a kit comprising such a dispensing unit, with which kit it is possible to monitor whether a predetermined procedure is correctly followed.

It is a further object of the present invention to provide an alternative to the prior art.

In particular, it may be seen as an object of the present invention to provide a dispensing unit that solves the above-mentioned problems of the prior art.

### SUMMARY OF THE INVENTION

Thus, the above-described object and several other objects are intended to be obtained in a first aspect of the invention by providing a dispensing unit for dispensing a substance into an external container, the dispensing unit comprising:
- a substance storage having a rotational axis and comprising at least one compartment for storing the substance, each compartment comprising at least one compartment opening, and
- a connector part in mutual rotational engagement with the substance storage around the rotational axis, the connector part comprising at least one flow passage and at least one dispensing outlet,
- wherein the at least one compartment opening of the substance storage and the at least one flow passage of the connector part are arranged so that:
   - for at least one mutual rotational orientation of the connector part and the substance storage around the rotational axis, substance can flow from the at least one compartment, through the at least one flow passage, and into the external container via the dispensing outlet of the connector part, and
   - for at least one different mutual rotational orientation of the connector part and the substance storage, substance cannot flow through the at least one compartment opening, and
- wherein the substance storage and the connector part comprise mutually engagable first and second engagement means, respectively, which are designed and arranged to ensure that the mutual rotation of the connector part and the substance storage follows a predetermined sequence of mutual rotations between stable mutual rotational orientations.

For the presently preferred uses of the invention, such as those described in relation to the figures, the substance is a fluid. However, it could also be a solid material, such as a powder, provided that it is adapted to flow out of the compartments in the intended manner.

By "stable" is preferably meant that the components will not move unintendedly if no force is applied. Such unintended application of forces could e.g. be due to lack of attention if the user is disturbed, or it could be from the dispensing unit being dropped on the floor. The components will typically be maintained in the stable positions by the action of frictional forces which should be overcome by manually applying a rotational force to move the components from one stable position to the next. It would also be possible to use the dispensing unit in an automated or semi-automated process, wherein the rotational force is applied by external equipment in which the dispensing unit has been placed.

The external container may e.g. be suitable for holding a tissue sample as will be described in further details below.

In presently preferred embodiments, the connector part is at least partially surrounding the substance storage; examples will be given in relation to the figures. However, the geometries may alternatively or in combination therewith be so that the substance storage at least partially surrounds the connector part.

The first and second engagement means may be designed and arranged to enable the following predetermined sequence of stable mutual rotational orientations:
- a) no fluid communication between the at least one compartment and the at least one dispensing outlet,
- b) fluid communication between at least one of the at least one compartments and the at least one dispensing outlet, and
- c) no fluid communication between the at least one compartment and the at least one dispensing outlet.

By "fluid communication" is preferably meant that the substance, which may be a fluid or a solid material e.g. in powder form, can flow between the locations being in fluid communication.

Such a dispensing unit may further comprise a subsequent stable mutual rotational orientation, wherein there is fluid communication between at least one of the at least one compartments and the at least one dispensing outlet. Such a further mutual rotational orientation will be particularly relevant in relation to a kit according to the second aspect of the invention as will be described below. Here it can used to remove the substance back into the compartments by turning the kit upside-down so that the substance can be flow due to gravity. This will e.g. be advantageous in relation to a method of dispensing a reagent for preservation of tissue sample in accordance with the third aspect of the invention; see below. By using this option, the dispensing unit can be used for safe handling of the used reagent. The dispensing unit comprising the used reagent can then be transported to another place for handling of the possibly toxic fluid in a process which is safe with respect to both health and environment. If desired, the kit may comprise a quick release button to ensure that the described possibility of letting the substance return to the substance compartment can only be done on purpose.

In some embodiments of the invention, the dispensing unit further comprises a locking part having fastening means adapted to engage with the connector part, so that when the fastening means engages with the connector part, a fluid tight sealing is obtained between the at least one compartment the at least one dispensing outlet. This locking and tightening function could alternatively be incorporated into one of the other parts. The locking means can e.g. be threads, snap-lock, and mutually interlocking protrusions and indentations. As an example, the connection between the connector part and the substance container could contain these functions. Furthermore, one or more sealing components may be comprised in the dispensing units to further ensure a fluid tight connection being established. An example of a possible design of a sealing component will be shown in the figures. Sealing components can e.g. be made from materials such as polymers, elastomers or other materials which can form a sealing which prevents the substance to pass it. The sealing components are arranged between other parts of the dispensing unit to ensure that no flow of substance will occur unless intended.

In presently preferred embodiments of the invention, the mutual rotation of the connector part and the substance storage may be temporarily prevented by mutual engagement between first and second blocking means provided at corresponding positions of the substance storage and the connector part, respectively, and by the connector part comprising release means which must be activated, such as pressed or twisted, to interact with at least one of the first and second blocking means to enable the further mutual rotation of the substance storage and the connector part. With such embodiments, a higher safety against unintended mutual rotation is obtained as will be further clarified in relation to the figures.

In some of the embodiments having first and second blocking means,
- the first blocking means are protrusions provided on an outer circumferential surface of the substance storage,
- the second blocking means are depressions, recesses or holes provided in an inner circumferential surface of the connector part, and
- the release means are tabs which can be pushed towards and elastically deform the substance storage to move the protrusions out of the engagement with the corresponding depressions, recesses or holes.

An example of such an embodiment is shown in some of the figures.

The connector part may comprise a lower connector part comprising the at least one flow passage and the at least one dispensing outlet, and an upper connector part connected with the lower connector part so that they rotate as one unit, when the dispensing unit is in an assembled state. By manufacturing the connector part by assembly of two sub-parts instead of as one unit, the manufacturing may at least for some geometries be significantly more cost-effective. A possible further advantage is that it will hereby be possible to customize the connector part to allow for different used of the dispensing unit by making a part of the connector part exchangeable to meet specific needs and predetermined sequences.

The substance storage may comprise a storage compartment part comprising the at least one compartment for storing the substance, and a storage engagement part comprising the first engagement means and connected with the storage compartment part so that they rotate as one unit, when the dispensing unit is in use. The connection may e.g. be due to geometrical constraint, such as the storage compartment part having a geometry which can be fitted within the storage engagement part in a mutually non-rotational manner. Alternatively or in combination therewith, the parts may be connected by other means, such as by press fit or snap-lock, or by other mutually engaging protrusions and depressions. Some examples will be shown in the figures.

Certain biological procedures, such as water sample analysis, may involve dispensing of different fluids that need to be dispensed in accurate amounts and in a certain order, and may furthermore involve components as inhibitors and toxins. These analyses are therefore time consuming and may even lead to unsuccessful results due to incorrectly followed procedures; they may additionally constitute a risk for the person performing the analysis. For this type of analyses it would therefore be advantageous to obtain a dispensing device that is easier to use and with a lower risk of incorrectly followed procedures, including using incorrect amounts of fluids.

The dispensing unit may also be used for sample collection and sample analysis within other technical areas, such as within the chemical, petro-chemical or food industries. Here it is a known problem that samples are often taken out by nonprofessionals who do not always have enough experience with how to correctly follow prescribed procedures. For such uses, it will be an advantage to be able to design the dispensing unit to precisely match the actual need and so that it can only be used in the intended way and without any contamination of the sample. For some uses, it may even be possible to design the unit to contain all the chemicals needed for a specific experiment facilitating the performance of experiments outside a laboratory.

The substance storage may comprise two or more compartments for storing one or more type of substance. There may e.g. be 2 to 18 compartments, such as from 2 to 10 or from 5 to 18, such as from 2 to 5 or from 5 to 10 or from 10 to 15. In the same manner, the external container may also comprise more than one compartment, such as the same numbers as given for the compartments. If desired or necessary, the flow of substance from the compartments and into the external container may be assisted by applying vacuum to the external container. This may be particularly relevant for embodiments having so small volumes in the compartments that it may be difficult at least for some substances to make them flow by gravity only.

Furthermore, the substance storage and the connector part may be designed and arranged so that it is possible to dispense substance from one compartment at a time. This may e.g. be relevant for uses of the device for which an analysis, such as analysis of a biological or chemical sample, requires that there is a period of time between dispensing fluids from the different compartments, or if two or more fluids may not be mixed before use and/or has to be dispensed in a particular order. Such need for dispensing from one compartment at a time will be taken into account when designing the arrangement of the compartment openings of the two or more compartments and the at least one flow passage of the connector part. Furthermore, if the embodiment comprises blocking means, the design and arrangements thereof will also have to be taken into account in order to meet the design requirements. The determination of the specific design to use for a given application of the dispensing unit will typically be based on computer simulations, possibly combined with additive manufacturing, such as 3D printing.

Some embodiments may be designed so the between steps of dispensing substance from two different compartments, there is a step of removing the first substance from the external container back into the substance storage, before the next substance is dispensed. Hereby it will be possible to have the sample exposed to one substance at a time in a predetermined order.

Each of the substance storage, the connector part, and the possible further components as described above and below may be made of one or more of the following materials: polymer, glass, metal or ceramic. If one or more of the parts is/are made of metal, it could e.g. be stainless steel or any metal alloy that can serve as a container for a fluid. For some materials, it will be possible to use transparent material. Hereby it can be visually inspected that the dispensing unit is appropriately assembled, including e.g. possible sealing components, so that a fluid tight sealing is ensured. It may also be advantageous to be able to check visually e.g. that all the substance has been dispensed and that there is no leak e.g. due to a worn part. For some applications, it may be required to use a material which can be sterilized. It may furthermore be required to use a material which can withstand endothermic and/or exothermic processes.

The substance storage may have at least one filling opening through which substance can be filled into the at least one compartment, preferably wherein the at least one filling opening is provided with a lid. Such at least one filling opening may e.g. be arranged at an upper end of the substance storage, but it could also be placed at other positions depending on the process used to fill the compartments. The filling may be done at an external location, such as a factory, or it may be done locally close to the location of use of the dispensing unit. It may e.g. be desirable to be able to fill the dispensing unit locally when it is used for research so that only a few dispensing units are used and so that they can be filled with a substance dependent on an actual study to be performed. An alternative to having a lid could be some other kind of cover, possibly not removable after closure. The at least one filling opening can also be used to remove the substance as will be explained in further details below.

In some embodiments of the invention, the substance storage may have at least two compartments, and may have at least one wall separating two compartments which at least one wall is breakable in response to a mutual rotational movement of the connector part and the substance storage. This functionality can e.g. be obtained by the wall collapsing due to deformation taking place in response to the mutual rotation of the connector part and the substance storage. It may alternatively or in combination therewith be obtained by the wall being pierced by some kind of piercing means which is activated, such as moved, in response to the rotational movement. Embodiments having such breakable walls between the compartments may e.g. be used for applications where two substances are to be mixed only shortly before use. By use of the present invention, it will hereby be possible to have to two substances available in the prescribed amounts and ready for use whereby a device is obtain which is easy and safe to use.

In any of the embodiments as described above, the connector part preferably comprises fastening means for fastening it to the external container. Such fastening means may e.g. be thread connections which allow for an easy assembly. It could alternatively be snap-lock, press fit or another kind of locking mechanism.

In some embodiments of the invention, the dispensing unit has a reagent for preservation of tissue samples stored in the at least one compartment. At least for some applications, it will be important to ensure that the substance storage containing enough substance to fill the whole of the external container, such as having 10% more substance than the volume of the external container. Hereby it can be ensured that the external container is completely filled. This will e.g. be relevant in relation to samples that would otherwise degrade by unintended exposure to the surrounding environment. For use of the dispensing unit in relation to water samples for some kinds of analysis, a specific amount of reagent should be added to perform the analysis. In such uses, the volume of the compartment may be the same or larger than the needed volume of the reagent stored therein; i.e. it is not necessary that the compartments are full.

In a second aspect, the present invention relates to a kit comprising an external container and a dispensing unit as described above, wherein the external container and the connector part are mutually connectable or connected. The connection is typically a thread connection, but it may also be another type of connection, such as a snap-fastening or press-fit.

For higher safety against unintended, such as too early, disconnection between the external container and the dispensing unit, the external container and the connector part may comprise mutually engagable safety means designed and arranged so that when the external container has been connected with the connector part, it is prevented from being disconnected again until after a predetermined part of the sequence of mutual rotations has been performed. Such safety means may e.g. be corresponding protrusions and indentations or other mutually interlocking geometries, such as screw threads. The actual type of safety means is determined as part of the design process.

The external container may comprise tracking and monitoring means, such as a RFID, QR code, 3D code and other means of smart labelling and tracking. Other sensors and means can be used to enable data collection and monitoring of the samples and reagents dependent on an actual use of the dispensing unit. Such tracking and monitoring means may be adapted to be activated by the mutual movement between the connector part and the substance storage. This can e.g. be obtained by the connector part comprising activation protrusions which are moved into engagement with the tracking and monitoring means during the rotation. By incorporating such tracking and monitoring means, it will be possible e.g. to store information about exactly when the substance was transferred to the external container. This can be important e.g. to be able to monitor, and possibly guarantee, that the sample in the external container has been exposed to the substance for a prescribed amount of time. For many uses of the dispensing unit, it will also be relevant to measure and monitor the temperature to which the dispensing unit and the samples have been exposed. This is particularly relevant for analysis and samples which have to be kept within predetermined temperature limits. For embodiments having more than one type of substance which is to be applied to the sample in a prescribed sequence, it will also be possible to monitor that this is done. Hereby it will be possible for the analyst to know how the sample has been handled, including the timing of the different steps of the process. This can make it easier to perform a diagnosis since a possible undesired influence from other variables than those to be studied can be avoided.

The external container may further comprise partition walls e.g. to store more than one sample in the external container, which samples are to be kept separated e.g. to avoid damage to the samples.

A kit according to the invention may further comprise at least one sample placement means, such as a filter, arranged between the connector part and the external container. Such a filter can be used for easy retrieval and adding of the sample without damaging it. This may e.g. be further facilitated by using a filter which is bag-shaped so that it is easy to grip by e.g. by a tool used to remove the sample. It will also be possible to have more than one filter arranged, preferably on top of each other. If the filters have different mesh sizes, one of them can be used for easy removal of e.g. debris and another for removal of the sample itself. Filters having different sizes can also be used for sorting of multiple samples according to size.

Some embodiments of the kits may comprise an internal edge inside the external container at a distance from an upper edge of the external container. Such an edge may facilitate the placement of the sample in the external container, such as for removing it from e.g. a blade of a knife used to obtain the sample. This will be particularly relevant if the external container is held by one hand and a tool with the sample is held by the other hand. The edge may e.g. be in the form of a component containing a slit through which the tool, such as the blade of a knife can be inserted; one side of such a slit may be considered as an edge.

In a third aspect, the invention relates to a method of dispensing a substance into an external container comprising a sample, the method comprising the use of a kit as described above, and the method further comprising the following steps:
- placing the sample in the external container,
- connecting the external container to the connector part,
- mutually rotating the connector part and the substance storage around the rotational axis, to allow substance to flow from the at least one compartment and into the external container via the dispensing outlet of the connector part, and
- optionally mutually rotating the connector part and the substance storage to close the compartment opening so that substance cannot flow from the at least one compartment through the at least one compartment opening, through the at least one flow passage, and into the at the external container via the dispensing outlet of the connector part,
- optionally transporting the dispensing unit containing the sample to another location,
- disconnecting the external container from the connector part, and
- removing the sample from the external container.

With such a method, the steps are typically to be performed in the same order as mentioned above. This means that the dispensing unit is preferably designed so that the external container cannot be disconnected from the connector part until at the end of the predetermined sequence of mutual rotations between stable mutual rotational orientations.

By "mutually rotating" and "mutually rotate" two parts is here and in the rest of text preferably meant that the mutual orientation of the two parts is changed. This can be done by rotating both parts or rotating one of them while the other is not rotated.

Such a method may further comprise the following steps before the step of disconnecting:
- moving the kit into a spatial orientation which allows the reagent to flow from the external container and back into at least one compartment of the substance storage, and
- optionally mutually rotating the connector part and the substance storage to close the at least one compartment opening so that substance cannot flow through the at least one compartment opening.

The first, second and third aspects of the present invention may each be combined with any of the other aspects. These and other aspects of the invention will be apparent from and elucidated with reference to the embodiments described hereinafter.

### BRIEF DESCRIPTION OF THE FIGURES

The dispensing unit according to the invention as well as possible uses thereof will now be described in more detail with regard to the accompanying figures. The figures show one way of implementing the present invention and is not to be construed as being limiting to other possible embodiments falling within the scope of the attached claim set.
Figure 1 shows schematically a kit comprising a dispensing unit according to the present invention.
Figure 2 shows in exploded three-dimensional view the kit in figure 1.
Figure 3 shows a partial sectional view of the substance storage and the upper connector part of the kit in figure 2.
Figure 4 shows schematically and in cross-sectional view an alternative embodiment in which the substance storage comprises a storage compartment part and a storage engagement part.
Figure 5 shows schematically and in exploded three-dimensional view an alternative embodiment of the invention in which the connector part comprises an upper and a lower connector part.
Figure 6 shows the embodiment in figure 5 in an assembled condition.
Figure 7 shows schematically an embodiment having more compartments for storing substance.
Figure 8 shows schematically a method according to the invention.

### DETAILED DESCRIPTION OF AN EMBODIMENT

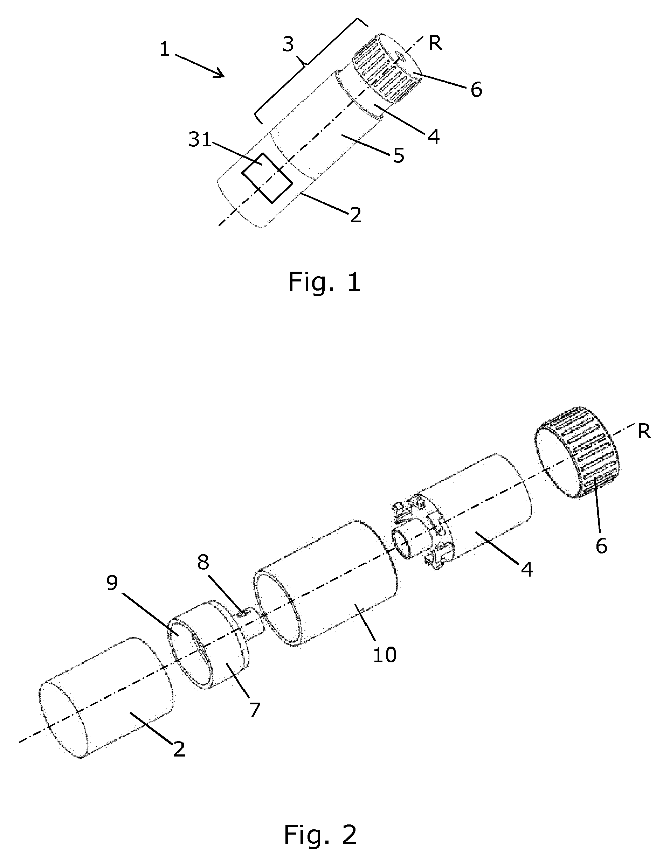

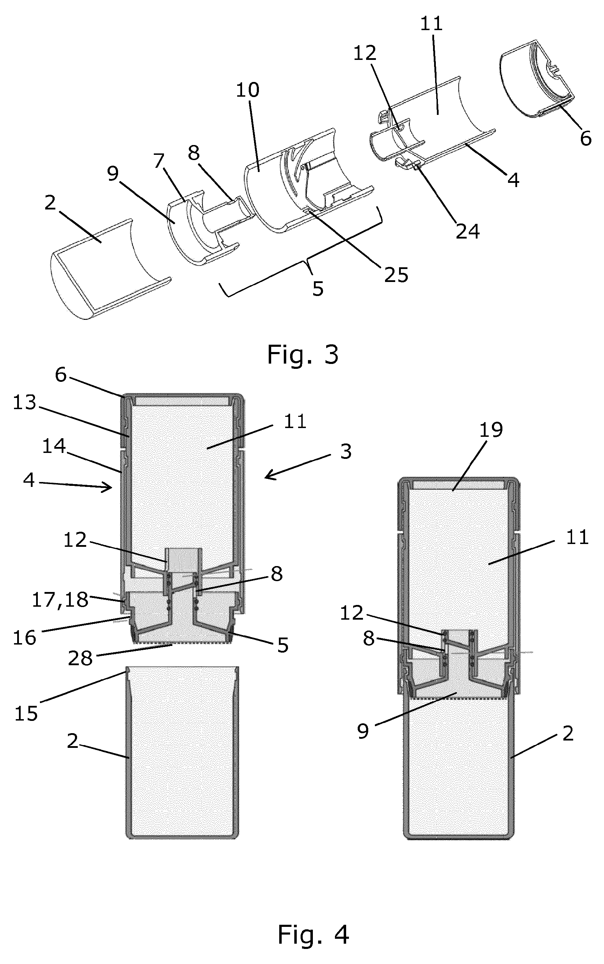

Figure 1 shows schematically a kit 1 comprising an external container 2 and a dispensing unit 3. The dispensing unit 3 comprises a substance storage 4 and a connector part 5 in mutual rotational engagement with the substance storage 4 around the rotational axis R. The substance storage 4 is provided with a lid 6. Figure 2 shows the kit in figure 1 in exploded three-dimensional view, and figure 3 shows the kit in figure 2 in a cross-sectional view along a longitudinal axis. The figures illustrate an embodiment in which the connector part 5 comprises a lower connector part 7 comprising the at least one flow passage 8 and the at least dispensing outlet 9, and an upper connector part 10 connected with the lower connector part 7 so that they rotate as one unit, when the dispensing unit 3 is in an assembled state. The lower and upper connector parts 7,10 may e.g. be made by injection moulding, and the parts may e.g. be assembled by ultrasound welding or other methods for locally melting the materials so that they are joined at the points where they touch. Other methods of assembly may also be used, such as press fit, snap-lock, or adhesion, such as gluing.

The substance storage 4 comprises at least one compartment 11 for storage of the substance to be dispensed. Figure 3 shows an embodiment having one compartment 11 only. The compartment 11 has a compartment opening 12 for outlet of the substance from the compartment 11. In the embodiment in figures 2 and 3, the connector part 7 comprises one flow passage 8 and one dispensing outlet 9. However, as explained above other embodiments of the invention comprise more than one flow passage and/or more than one dispensing outlet. The compartment opening 12 of the substance storage 4 and the flow passage 8 of the lower connector part 7 are arranged so that:
- for at least one mutual rotational orientation of the connector part 5 and the substance storage 4 around the rotational axis R, substance can flow from the compartment 11, through the flow passage 8, and into the external container 2 via the dispensing outlet 9 of the connector part 5, and
- for at least one different mutual rotational orientation of the connector part 5 and the substance storage 4, substance cannot flow through the compartment opening 12. This will be more clearly shown in relation to figure 4.

In the embodiment in figures 2 and 3, the compartment opening 12 and the flow passage 8 are located close to the rotational axis R. However, many other locations will be possible including having the compartment opening 12 located on a circumferential outer surface of the substance storage 4 and the flow passage 8 located on a surface of the connector part 5 surrounding the outer surface of the substance storage 4.

Figure 4 shows schematically and in cross-sectional view, an alternative embodiment in which the substance storage 4 comprises a storage compartment part 13 and a storage engagement part 14. The external container 2 is shown before assembly with the dispensing unit 3 in figure 4.a. It is pressed onto the connector part 5 by forcing a circumferential protrusion 15 on the upper edge of the external container 2 into engagement with an inner recess 16 on the external container 2 as shown in figure 4.b. The storage engagement part 14 is connected with the storage compartment part 13 so that they rotate as one unit, when the dispensing unit 3 is in use. Figure 4.a shows a closed position, wherein the mutual rotational orientation of the substance storage 4 and the connector part 5 are so that there is no flow of substance from the compartment 11. Figure 4.b shows an open position, wherein the connector part 5 has been turned 180° to an orientation where the compartment opening 12 and the flow passage 8 are aligned to allow substance to flow from the compartment 11 and into the external container 2 via the dispensing outlet 9.

As shown schematically in figure 4, the substance storage 4 and the connector part 5 comprise mutually engagable first and second engagement means 17,18, respectively. The first and second engagement means 17,18 are designed and arranged to ensure that the mutual rotation of the connector part and the substance storage follows a predetermined sequence of mutual rotations between stable mutual rotational orientations. The first and second engagement means 17,18 may e.g. be in the form of protrusions and grooves arranged and dimensioned to allow for the mutual rotational engagement. However, many other approximately mating geometries would be possible, such as ribs, tabs, pins, edges, and holes. In the embodiment in figure 4, the first engagement means 17 is the groove on the inner surface of the storage engagement part 14 of the substance storage 4. The second engagement means 18 is the circumferential protrusion on the connector part 5.

The substance storage 4 in the embodiment in figure 4 has a filling opening 19 through which substance can be filled into the compartment 11. The filling opening 19 is provided with a lid 6 for closure of the filling opening 19. Other ways of closing will also be possible, the one chosen for a given application depending on whether or not it the opening should also be used for later removal of substance. This may e.g. be relevant in relation to a method of use as described above, where the used substance is returned back into the compartment for safe and easy handling of possibly toxic reagent.

In a typical embodiment, the first and second engagement means 17,18 are designed and arranged to enable the following predetermined sequence of stable mutual rotational orientations:
- a) no fluid communication between the at least one compartment 11 and the at least one dispensing outlet 9,
- b) fluid communication between at least one of the at least one compartments 11 and the at least one dispensing outlet 9, and
- c) no fluid communication between the at least one compartment 11 and the at least one dispensing outlet 9.

Optionally, the design of the first and second engagement means 17,18 may allow for a subsequent stable mutual rotational orientation, wherein there is fluid communication between at least one of the at least one compartments 11 and the at least one dispensing outlet 9.

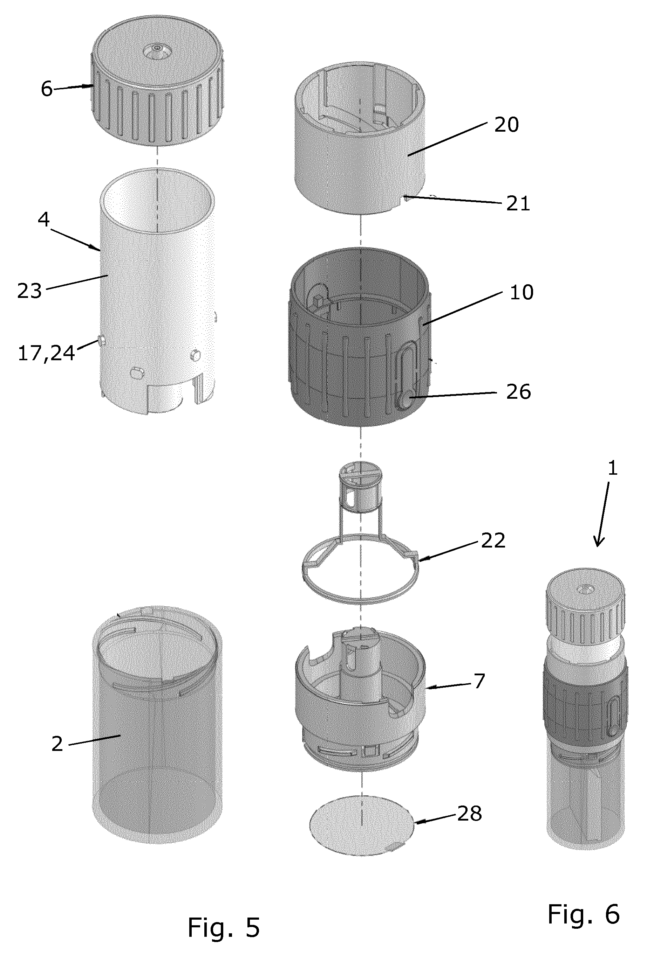

Figure 5 shows schematically and in exploded three-dimensional view an alternative embodiment of the invention in which the connector part 5 comprises an upper connector part 10 and a lower connector part 7. Figure 6 shows the embodiment in figure 5 in an assembled condition. This embodiment further comprises a locking part 20 having fastening means 21 adapted to engage with the connector part 5, so that when the fastening means 21 engages with the connector part 5, a fluid tight sealing is obtained between the at least one compartment 11 the at least one dispensing outlet 9. The dispensing unit preferably also comprise sealing components, such as O-rings. An example of a possible design of a sealing component 22 designed for a specific design of the dispensing unit is shown schematically in figure 5. In the embodiment in figure 5, the substance storage 4 is one unit and comprises the first engagement means 17 provided on an outer circumferential surface 23 in the form of protrusions. The corresponding second engagement means of the connector part 5 are not shown in this figure but will e.g. be in the form of a recess on an inner surface of the upper connector part.

A dispensing unit 3 according to the invention may preferably be designed so that the mutual rotation of the connector part 5 and the substance storage 4 can be temporarily prevented by mutual engagement between first and second blocking means 24,25 provided at corresponding positions of the substance storage 4 and the connector part 5, respectively. Such blocking means may e.g. be in the form of mating protrusions 24 and holes 25 as shown schematically in the embodiment in figure 3. Here the first blocking means 24 are protrusions provided near the lower end of the substance storage 4, and the second blocking means 25 are holes provided in an inner circumferential surface of the upper connector part 10. In the embodiment in figure 5, the first blocking means 24 in the form of protrusions also form the first engagement means 17. The second blocking means may be holes arranged in a surface of grooves, these grooves forming second engagement means. However, other geometrical combinations may also fulfil the described functioning.

The release of the temporary blocking obtained thereby to enable the further mutual rotation of the connector part 5 and the substance storage 4 can be ensured by the connector part 5 comprising release means 26 which must be activated, such as pressed or twisted, to interact with at least one of the first and second blocking means 24,25. In the embodiment in figure 5, the release means 26 are tabs which can be pushed towards and elastically deform the substance storage 4 to move the protrusions 24 out of the engagement with the corresponding depressions, recesses or holes 25. The activation of the release means 26 typically involve forces being applied by the user. Furthermore, the geometry of e.g. a recess may also be so that a twist applied to the parts force the blocking means in a desired direction.

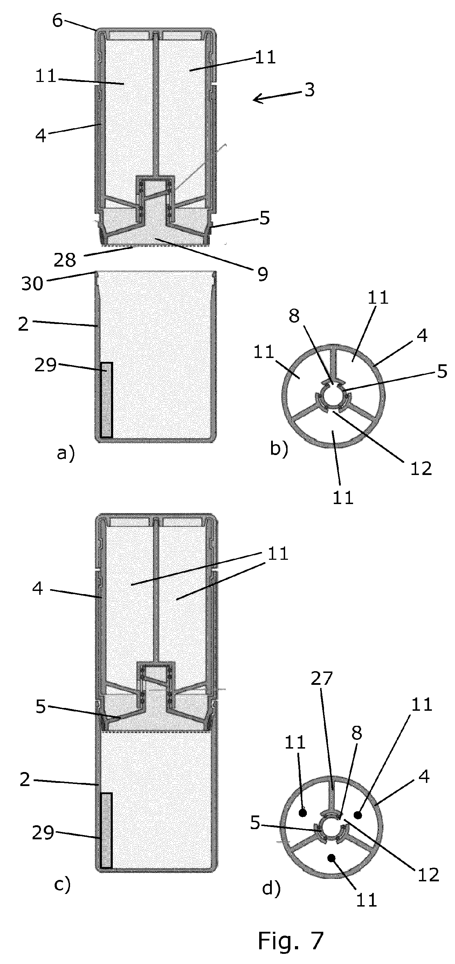

The substance storage 4 may comprises two or more compartments 11 for storing one or more type of substance. An example of such an embodiment having three compartments 11 is shown schematically in figure 7. This embodiment resembles the one in figure 4 with respect to the other features. Figure 7.a shows the dispensing unit 3 before assembly with the external container 2. Figure 7.b shows a cross-sectional view at the central location of the connection between the substance storage 4 and the connector part 5 in figure 7.a and illustrates that there is no fluid connection between the compartment 11 and the dispensing outlet 9. Figure 7.c shows the external container 2 assembled with the connector part 5 and with the substance container 4 and the connector part 5 in a mutual rotational orientation where there is fluid communication between one of the compartments 11 and the external container 2. This fluid connection is seen in figure 7.d which shows a cross-sectional view at the central location of the connection between the substance storage 4 and the connector part 5 in figure 7.c.

In such dispensing units 3with two or more compartments 11, the substance storage 4 and the connector part 5 may be designed and arranged so that it is possible to dispense substance from one compartment 11 at a time. For such an embodiment wherein the substance storage 4 has at least two compartments 11, at least one wall 27 separating two compartments 11 may be breakable in response to a mutual rotational movement of the connector part 5 and the substance storage 4.

A kit 1 according to the invention may further comprise one or more filters 28 arranged between the connector part 5 and the external container 2 as shown schematically in figures 4, 5 and 7. Such a filter 28 may facilitate the removal of the sample from the external container 2 without damaging it. At least for some kind of samples, it may be particularly advantageous if the filter 28 in the form of a bag arranged in the external container 2 before the sample is placed therein. This may e.g. be useful when the sample can be difficult to handle. In that case, it can be handled by holding the filter 28.

At least for some kinds of samples, such as thin slices of tissue samples, it can be difficult to place the sample in the external container 2 without damaging it, because it is difficult to remove the sample from the knife or other tool used to obtain it. For such uses, the external container 2 can advantageously be provided with an internal edge 29 inside the external container 2 at a distance from an upper edge 30 of the external container 2; see figure 7. Such an internal edge 29 can e.g. be established as part of the manufacturing of the external container 2, such as by injection moulding. It can alternatively be established by placing a sheet of material, such as polymer foam along an edge of the inner surface of the external container.

For some embodiments of the invention, the external container comprises tracking and monitoring means 31, such as a RFID; this is shown schematically in figure 1. As explained above, such tracking and monitoring means 31 may be adapted to be activated by the mutual movement between the connector part 5 and the substance storage 4 to enable easy monitoring of how the dispensing unit 3 has been used.

A dispensing unit 3 as described above may be made in different sizes. For applications within preservation of tissue samples, such as skin samples, typical diameters will be 20-100 mm, such as 20-50 mm or 50 to 100 mm. The height of an assembled dispensing unit will typically be in to order of 50 to 150 mm, such as 50 to 100 mm or 100 to 200 mm. The total volume of the compartments will typically be in the order of 1 to 250 ml, such as 50 to 100 ml when the dispensing unit is used in relation to preservation of tissue samples. However, other dimensions are also covered by the scope of the present invention.

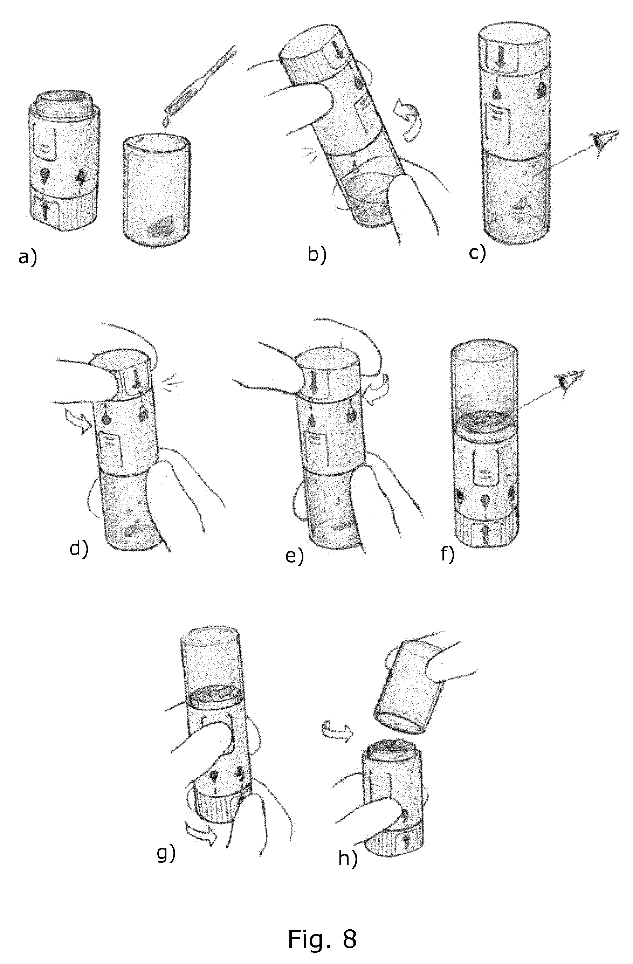

An example of a possible use of a kit as described above would be for dispensing a reagent for preservation of tissue sample placed in the external container. This could e.g. be relevant for tissue samples taken out of a body during a surgery, the samples to be subsequently analysed at another location. An example of a method of using the kit is shown schematically in figure 9. The kit would typically be used in the following manner:
- the tissue sample is placed in the external container; see figure 8.a
- the external container is connected to the connector part, and
- the connector part and the substance container are mutually rotated around the rotational axis, to allow the reagent to flow from the compartment and into the external container via the dispensing outlet of the connector part to cover the tissue sample; see figure 8.b. The external container is typically made from a transparent material so that it can be visually inspected that the tissue sample is fully covered by reagent; see figure 8.c. The amount of reagent should preferably be large enough to ensure that the tissue sample is fully covered to prevent any undesired chemical reaction due to exposure to the surrounding environment. The connector part and the substance storage can then be mutually rotated to close the compartment opening so that any remaining reagent cannot flow from the at least one compartment; see figure 8.d. Typically, the dispensing unit containing the sample is then transported to another location for further handling and analysis. When at the right location, the external container is removed from the connector part, and the tissue sample is removed from the external container. If desired, the dispensing unit can furthermore be used for storage and handling of the used reagent. This will be possible by mutually rotating the substance storage and the connector part to open the flow passage between the external container and at the least one compartment, see figure 8.e, and holding the kit in a spatial orientation which allows the reagent to flow from the external container and back into the substance storage; this is shown in figure 8.f. When the reagent has flown back into the container, the connector part and the substance storage can be mutually rotated to close the compartment opening so that the reagent is kept inside the compartment; see figure 8.g. After the predetermined sequence of mutual rotations has now been followed, the external container can be removed from the dispensing unit as shown in figure 8.h. The kit in figure 8 comprises a filter, and as shown in figure 8.h this results in the tissue sample ending on that filter for easy removal.

The use of a dispensing unit according to the invention has with reference to the figures been described in relation to dispersion of a reagent onto tissue samples. However, the invention may be used for a number of other applications. Some examples have been described in the section summary of the invention. For some of these examples it may not be relevant to include the steps of letting the substance flow back into the at least one compartment. It may e.g. be the case for liquid samples which mix with the substance so that further analysis is to be performed on the mixture.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. Dispensing unit (3) for dispensing a substance into an external container (2), the dispensing unit (3) comprising:
- a substance storage (4) having a rotational axis (R) and comprising at least one compartment (11) for storing the substance, each compartment (11) comprising at least one compartment opening (12), and
- a connector part (5) in mutual rotational engagement with the substance storage (4) around the rotational axis (R), the connector part (5) comprising at least one flow passage (8) and at least one dispensing outlet (9),
- wherein the at least one compartment opening (12) of the substance storage (4) and the at least one flow passage (8) of the connector part (5) are arranged so that:
- for at least one mutual rotational orientation of the connector part (5) and the substance storage (4) around the rotational axis (R), substance can flow from the at least one compartment (11), through the at least one flow passage (8), and into the external container (2) via the dispensing outlet (9) of the connector part (5), and
- for at least one different mutual rotational orientation of the connector part (5) and the substance storage (4), substance cannot flow through the at least one compartment opening (12), and
- wherein the substance storage (4) and the connector part (5) comprise mutually engagable first and second engagement means (17,18), respectively, which are designed and arranged to ensure that the mutual rotation of the connector part (5) and the substance storage (4) follows a predetermined sequence of mutual rotations between stable mutual rotational orientations.

2. Dispensing unit (3) according to claim 1, wherein the first and second engagement means (17,18) are designed and arranged to enable the following predetermined sequence of stable mutual rotational orientations:
- a) no fluid communication between the at least one compartment (11) and the at least one dispensing outlet (9),
- b) fluid communication between at least one of the at least one compartments (11) and the at least one dispensing outlet (9), and
- c) no fluid communication between the at least one compartment (11) and the at least one dispensing outlet (9).

3. Dispensing unit (3) according to claim 2, comprising a subsequent stable mutual rotational orientation, wherein there is fluid communication between at least one of the at least one compartments (11) and the at least one dispensing outlet (9).

4. Dispensing unit (3) according to any of the preceding claims, further comprising a locking part (20) having fastening means (21) adapted to engage with the connector part (5), so that when the fastening means (21) engages with the connector part (5), a fluid tight sealing is obtained between the at least one compartment (11) the at least one dispensing outlet (9).

5. Dispensing unit (3) according to any of the preceding claims,
- wherein the mutual rotation of the connector part (5) and the substance storage (4) can be temporarily prevented by mutual engagement between first and second blocking means (24,25) provided at corresponding positions of the substance storage (4) and the connector part (5), respectively, and
- wherein the connector part (5) comprises release means (26) which must be activated, such as pressed or twisted, to interact with at least one of the first and second blocking means (24,25) to enable the further mutual rotation of the connector part (5) and the substance storage (4).

6. Dispensing unit (3) according to claim 5, wherein
- the first blocking means (24) are protrusions provided on an outer circumferential surface (23) of the substance storage (4),
- the second blocking means (25) are depressions, recesses or holes provided in an inner circumferential surface of the connector part (5), and
- the release means are tabs which can be pushed towards and elastically deform the substance storage (4) to move the protrusions out of the engagement with the corresponding depressions, recesses or holes.

7. Dispensing unit (3) according to any of the preceding claims, wherein the connector part (5) comprises:
- a lower connector part (7) comprising the at least one flow passage (8) and the at least one dispensing outlet (9), and
- an upper connector part (10) connected with the lower connector part (7) so that they rotate as one unit, when the dispensing unit (3) is in an assembled state.

8. Dispensing unit (3) according to any of the preceding claims, wherein the substance storage (4) comprises:
- a storage compartment part (13) comprising the at least one compartment (11) for storing the substance, and
- a storage engagement part (14) comprising the first engagement means (17) and connected with the storage compartment part (13) so that they rotate as one unit, when the dispensing unit (3) is in use.

9. Dispensing unit (3) according to any of the preceding claims, wherein the substance storage (4) comprises two or more compartments (11) for storing one or more type of substance.

10. Dispensing unit (3) according to claim 9, wherein the substance storage (4) and the connector part (5) are designed and arranged so that it is possible to dispense substance from one compartment (11) at a time.

11. Dispensing unit (3) according to any of the preceding claims, wherein the substance storage (4) has at least one filling opening (19) through which substance can be filled into the at least one compartment (11), preferably wherein the at least one filling opening (19) is provided with a lid (6).

12. Dispensing unit (3) according to any of the preceding claims, wherein the substance storage (4) has at least two compartments (11), and wherein at least one wall (27) separating the two compartments (11) is breakable in response to a mutual rotational movement of the connector part (5) and the substance storage (4).

13. Kit (1) comprising an external container (2) and a dispensing unit (3) according to any of the preceding claims, wherein the external container (2) and the connector part (5) are mutually connectable or connected.

14. Kit (1) according to claim 13, wherein the external container (2) comprises tracking and monitoring means (31), such as a RFID, QR code, or 3D code.

15. Kit (1) according to claim 14, wherein the tracking and monitoring means (31) are adapted to be activated by the mutual movement between the connector part (5) and the substance storage (4).

16. Kit (1) according to any of claims 13 to 15, further comprising at least one sample placement means (28), such as a filter, arranged between the connector part (5) and the external container (2).

17. Kit (1) according to any of claims 13 to 16, further comprising an internal edge (29) inside the external container (2) at a distance from an upper edge (30) of the external container (2).

18. Method of dispensing a substance into an external container (2) comprising a sample, the method comprising the use of a kit (1) according to any of claims 13 to 17, and the method further comprising the following steps:
- placing the sample in the external container (2),
- connecting the external container (2) to the connector part (5),
- mutually rotating the connector part (5) and the substance storage (4) around the rotational axis (R), to allow substance to flow from the at least one compartment (11) and into the external container (2) via the dispensing outlet (9) of the connector part (5), and
- optionally mutually rotating the connector part (5) and the substance storage (4) to close the compartment opening (12) so that substance cannot flow from the at least one compartment (11) through the at least one compartment opening (12), through the at least one flow passage (8), and into the at the external container (2) via the dispensing outlet (9) of the connector part (5),
- optionally transporting the dispensing unit (3) containing the sample to another location,
- disconnecting the external container (2) from the connector part (5), and
- removing the sample from the external container (2).

19. Method according to claim 18, the method comprising the following step before the step of disconnecting:
- moving the kit (1) into a spatial orientation which allows the reagent to flow from the external container (2) and back into at least one compartment (11) of the substance storage (4), and
- optionally mutually rotating the connector part (5) and the substance storage (4) to close the at least one compartment opening (12) so that substance cannot flow through the at least one compartment opening (12).

20. Method according to claim 18 or 19, wherein the substance is a reagent for preservation of tissue sample, and wherein the sample is a tissue sample.
